# EUROPEAN PATENT SPECIFICATION

(11) **EP 2 976 730 B1**
(45) Date of publication and mention of the grant of the patent: **11.08.2021**
(21) Application number: 14716643.3
(22) Date of filing: 18.03.2014
(51) Int. Cl.: G16H 40/63

(54) **AURAL ENHANCMENTS TO MEDICAL SYSTEMS**
AKUSTISCHE VERSTÄRKUNGEN FÜR MEDIZINISCHE SYSTEME
AMÉLIORATIONS SONORES DE SYSTÈMES MÉDICAUX

(30) Priority: 19.03.2013 US 201361803134 P
(43) Date of publication of application: 27.01.2016
(73) Proprietor: Koninklijke Philips N.V., 5656 AG Eindhoven (NL)
(72) Inventor: JANEVSKI, Angel, NL-5656 AE Eindhoven (NL); ZAGORCHEV, Lyubomir Georgiev, NL-5656 AE Eindhoven (NL)
(74) Representative: Zhu, Di
(86) International application number: PCT/IB2014/059914
(87) International publication number: WO 2014/147549

(56) References cited:
- EP-A2- 2 141 506
- US-A- 3 565 058
- US-A1- 2012 143 029

## Description

The present application relates generally to computer user interfaces. It finds particular application in conjunction with medical systems and will be described with particular reference thereto. However, it is to be understood that it also finds application in other usage scenarios and is not necessarily limited to the aforementioned application.

With the advent of technologies and broad data-delivery channels and applications, expert end-users in data-intensive domains increasingly rely on various visualization and quantification tools and methods to effectively navigate and interpret the data. For example, current tools and methods in radiology provide a high level of detail and present new opportunities to manipulate and characterize imaging data. In critical care, the overload of information may mask important alerts or delay the reaction time to actionable indicators. However, in a typical clinical setting, a radiologist is usually pressed for time. An interventional radiologist aims to optimize all information towards a most efficient procedure execution and outcome. In critical care, timely detection and prioritization of input is of essence for the clinical staff. When clinicians are overloaded with information, opportunities may be lost to use advanced quantification in their regular workflow. Methods and techniques that can enhance the perception of the clinician by providing quantitative information in a practical and useful way would be of great importance to the field. As another example, in other domains, such as meteorology and astronomy, similarly experts rely heavily on the analysis and presentation of data.

In the context of neurodegenerative disorders, rapid and accurate segmentation of multiple brain structures can be applied to patients and compared against a normative dataset. The normative dataset may contain information pertaining to, but not limited to, volume and shape for healthy control patients. Complimentary to structure volume, surfaces representing different brain structures can be used to describe the geometry of the structures' exterior. That enables the rapid calculation of morphometric statistics needed for objective quantitative comparison and diagnosis. A software system for rapid volumetric segmentation of brain regions coupled with a normative dataset provides: 1) the ability to identify specific structural abnormalities; 2) longitudinal monitoring of changes; 3) improved differential diagnosis; and/or 4) monitoring of the outcome of treatment or recovery. However, that information must be presented to the physician effectively by incorporating it into their normal workflow without compromising productivity. Having a separate standalone quantification system that provides printed reports is not often feasible and introduces a bottleneck in the standard workflow.

In addition to expert users, non-expert users are increasingly exposed to information delivered through multiple channels, such as GPS guidance while driving and smart phone notifications (e.g., e-mail, news, calls, etc.). Further, non-experts are increasingly interacting with these systems via voice commands, hands-on controls, and so on. Even in a more laid-back setting like watching television, users have increasingly more information to consider as there are increasing options available. For example, even while watching television, users are increasingly exposed to information to search for new programs, manage digital video recorder (DVR) recordings, visit web sites and services, or access on-demand streaming content.

One potential avenue for broadening the channels that deliver information to the end user is to use alternative and/or complementary senses, such as hearing. Typically, expert users that face the challenge of interpreting massive amounts of visual data operate in environments (e.g., operating rooms, radiology study rooms, etc.) where it should be easy to add aural signals to delivered images and image annotations. Further, non-expert users are typically already receiving audio notifications via some channel (e.g., GPS directions, smart phone notifications, etc.).

The use of audio can enrich computer user interfaces (UIs) at various levels. At the highest level, humans perceive sound or silence. Sound is classified as speech or non-speech sounds. Music is one of the more specific non-speech sound groups. Silence can be used as effectively as sound to mark boundaries, indicate a state, or limit revealing (i.e., purposely omitting the audio component of an entity in order to achieve altered perception of its power and state). The majority of approaches to sound-enhancement are implemented as auditory icons and earcons. With auditory icons, realistic or abstract sounds are mapped to events and interactions with entities in the UI. Auditory icons are recognizable sounds learned through experience and are used to identify the source of the sound. They can be used as direct representations of entity properties, but also as abstract or semi-abstract mappings. Earcons are generally not natural sounds. Earcons are built from simple components and/or motives and used to build structured sets of sounds (e.g., hierarchies) that are mapped to a particular aspect of the UI.

U.S. Patent No. 4,818,938 to Satin et al. discloses an approach to sound-enhancement for medical imaging. Two image representations of an interior region of a subject are generated. A first one of the image representations is displayed, and the other image representation is used to modulate an audio icon.

In US 2012/0143029 A1 a guidance system for assisting with the insertion of a needle into a patient body is disclosed. The guidance system utilizes ultrasound imaging or other suitable imaging technology. In one embodiment, the guidance system comprises an imaging device including a probe for producing an image of an internal body portion target, such as a vessel. One or more sensors are included with the probe. The sensors sense a detectable characteristic related to the needle, such as a magnetic field of a magnet included with the needle. The system includes a processor that uses data relating to the sensed characteristic to determine a 3-D position of the needle. The system includes a display for depicting the position of the needle. In other embodiments, a guidance system for guiding rigid or other medical instruments is disclosed, together with various example implementations thereof.

Further prior art can be found in US 4,818,938.

The present application provides a new and improved system, processor and non-transitory computer readable medium which overcome the above-referenced problems and others.

In accordance with the present invention, a medical system, a processor and a non-transitory computer readable medium are presented as defined in the claims.

One advantage resides in increased effectiveness of visualization and information delivery.

Another advantage resides in a reduced need to shift focus from visual perception mode to another mode (e.g., interpretation of numerical or other quantitative or visual results).

Another advantage resides in an additional channel to deliver alerts and other indicators.

Another advantage resides in an effective tool to quickly prioritize data subsets.

Another advantage resides in a safety mechanism that ensures user engagements (e.g., in cases of overwhelming data and/or user sensor fatigue).

Another advantage resides in a novel user interface

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.
FIGURE 1 illustrates a sensory generator system.
FIGURE 2 illustrates a definition of a frequency modulation (FM) based pattern.
FIGURE 3 illustrates a definition of a sine based pattern.
FIGURE 4 illustrates a definition of a FM based pattern.
FIGURE 5 illustrates a definition of a sine based pattern.
FIGURE 6 illustrates a definition of an envelope.
FIGURE 7 illustrates a definition of a rule for conveying density.
FIGURE 8 illustrates a definition of a rule for conveying change in position.
FIGURE 9 illustrates a medical system enhanced with the sensory generator system of FIGURE 1.
FIGURE 10 illustrates enhancements in radiology applications for conveying data regarding images and/or maps.
FIGURE 11 illustrates pseudo-code specifically instantiating the rule of FIGURE 7.
FIGURE 12 illustrates pseudo-code in which all rules and patterns that match a current context are instantiated.
FIGURE 13 illustrates a segmented image and/or map of a brain.
FIGURE 14 illustrates pseudo-code instantiating rules indicating change in position.
FIGURE 15 illustrates changing pitch through change in position of an image and/or map.
FIGURE 16 illustrates an X-ray fluoroscopy projection.
FIGURE 17 illustrates a multislice computed tomography (MSCT) image and/or map.
FIGURE 18 illustrates a surface representation of a deformable brain model.
FIGURE 19 illustrates a deformable brain model adapted to a patient's brain.
FIGURE 20 illustrates a flow chart for aurally conveying deviations between brain structures of a patient and normative brain structures.
FIGURE 21 illustrates a medical system enhanced with the sensory generator system of FIGURE 1.

Medical systems traditionally interface with users with the sense of sight. The present application enhances medical systems by using an additional sense, such as hearing, taste, smell, or touch, to interface with users. For example, when displaying images and/or maps of patients to users, data regarding the displayed images and/or maps is presented to the users using an additional sense, such as hearing and/or touch. This data includes, for example, alerts, spatial orientation of the images and/or maps, properties of relevant parts of the images and/or maps, properties of data that is not visually available to the user (e.g., neural substrates, metabolites, etc.), data reinforcing user actions in a particular context, deviations from normative datasets, and so on.

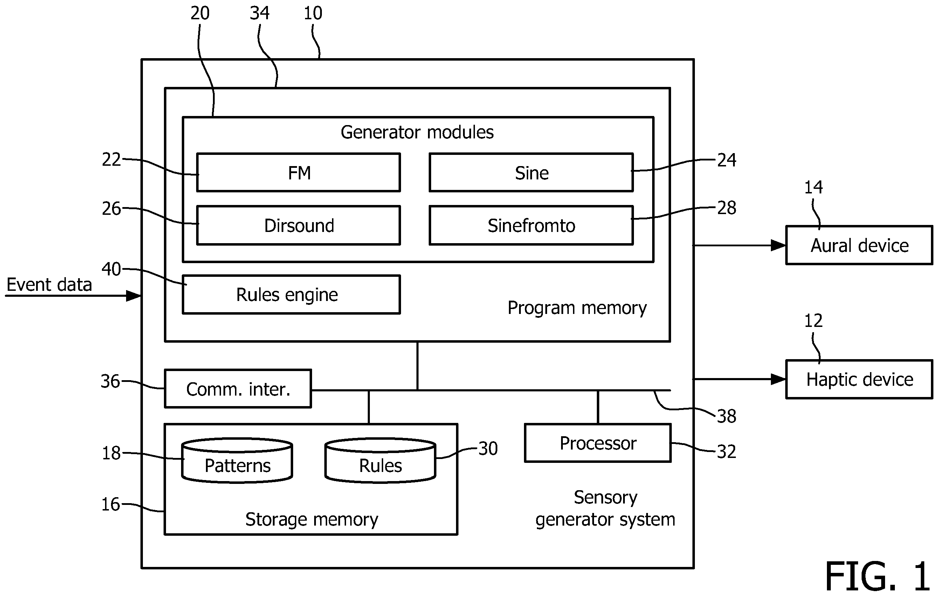

With reference to FIGURE 1, a sensory generation system 10 to enhance medical systems is provided. The sensory generation system 10 generates sensory signals, such as aural signals, for users of the sensory generation system 10. Sensory signals are signals to control devices invoking senses, such as hearing, taste, smell, sight and touch. Such devices include, for example, a haptic device 12, an aural device 14 (e.g., a speaker), and the like.

The sensory generation system 10 includes a storage memory 16 including a pattern database 18. The pattern database 18 includes one or more patterns for generating the sensory signals. Each of the patterns includes parameters defining a signal. Further, each of the patterns can be annotated with metadata to define the types of data to which the pattern can interface and/or to best match and identify the patterns from rules, discussed hereafter.

Each of the patterns further defines the type of signal to which the parameters pertain. Signal types include, for example, frequency modulation (FM), sine wave, and so on. The signal types are generated by a plurality of different software generator modules 20, such as an FM module 22, a sine module 24, a DIRSOUND module 26, and a SineFromTo module 28. Hence, each pattern can, for example, identify the type of signal by a link to one of the generator modules **20.**

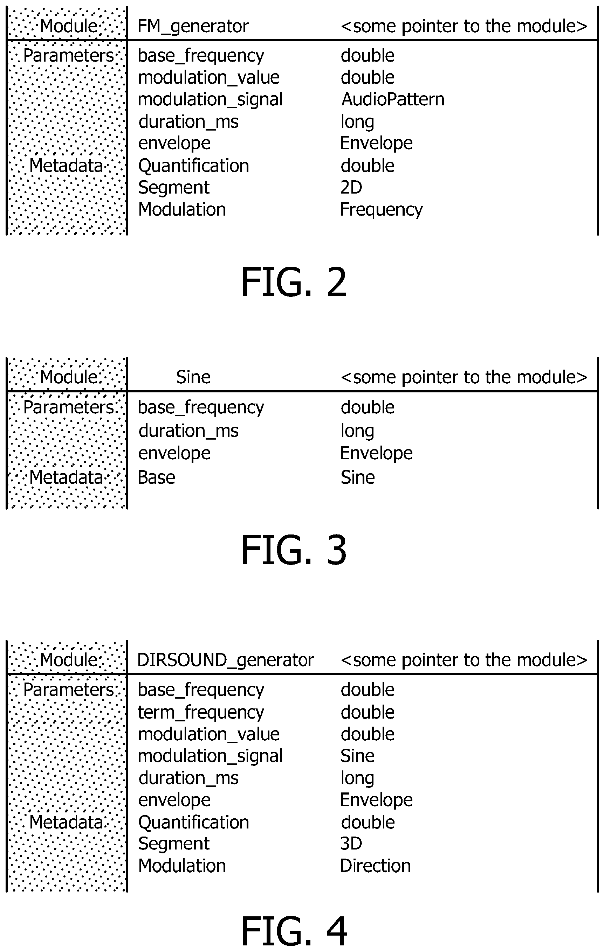

With reference to FIGURE 2, an example definition of a frequency modulation (FM) based pattern, identified hereafter as FM, is illustrated. FM includes a link to the FM module **22,** a plurality of parameters to control the FM module **22,** and metadata. Further, FM includes a modulation signal pattern, which points to another pattern, AudioPattern, as well as an envelope parameter. An example definition of AudioPattern is illustrated in FIGURE 3. AudioPattern includes a link to the sine module **24,** a plurality of parameters to control the sine module **24,** and metadata. Further, AudioPattern includes an envelope parameter.

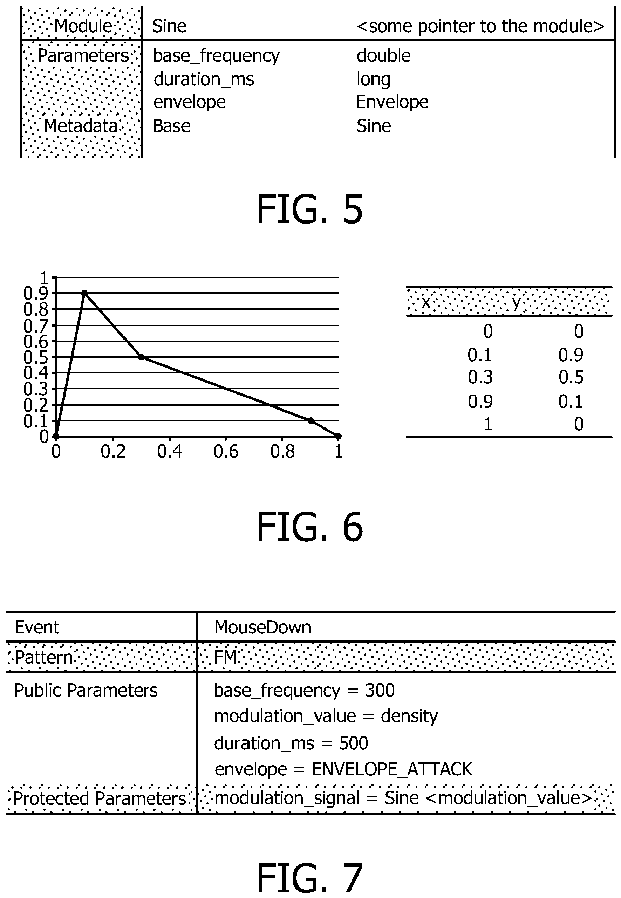

With reference to FIGURE 4, an example definition of a FM based pattern, identified hereafter as DIRSOUND, is illustrated. DIRSOUND includes a link to the DIRSOUND module **26,** a plurality of parameters to control the DIRSOUND module **26,** and metadata. Further, DIRSOUND includes a modulation signal pattern, which points to another pattern, Sine, as well as an envelope parameter. An example definition of Sine is illustrated in FIGURE 5. Sine includes a link to the SineFromTo module **28,** a plurality of parameters to control the SineFromTo module **28,** and metadata. Further, Sign includes an envelope parameter.

In view of the examples of FIGURES 2-5, parameters of the patterns can point to other patterns and/or define envelopes. An envelope can be described with a sequence of (x, y) value pairs to describe a two-dimensional (2D) curve in a 0-1 square space. The generated sound is then enveloped using this curve. An example definition of an envelope is described in FIGURE 6.

Referring back to FIGURE 1, the storage memory **16** further includes a rules database **30.** The rules database **30** includes one or more rules linking events, such as application and/or interface events, to the patterns. The rules are driven by the current application context. Each of the rules identifies an event and a pattern to link to the event. Further, the rule includes one or more parameters needed to instantiate the pattern. It is also contemplated that the rule can include parameters to define a loop for the pattern.

With reference to FIGURE 7, an example definition of a rule, identified hereafter as AudioDensityMouseDown, is illustrated. AudioDensityMouseDown builds on the examples of FIGURES 2 and 3. AudioDensityMouseDown is defined for a MouseDown event and specifies the parameters to instantiate FM, each parameter being public or protected. Further, the modulation_value parameter of AudioDensityMouseDown is set to a density parameter, which must be provided to execute the rule.

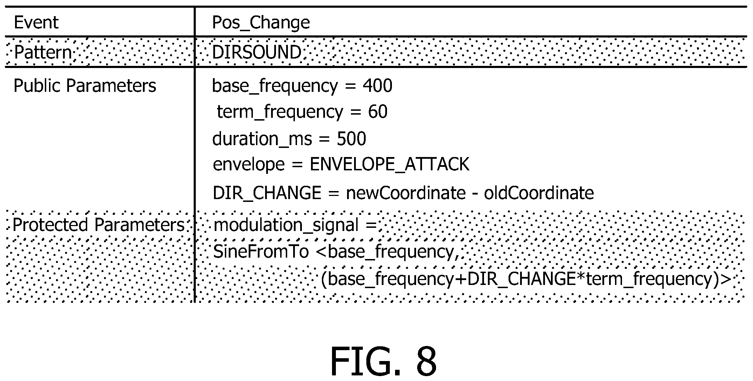

With reference to FIGURE 8, an example definition of a rule, identified hereafter as AudioDirectionChangeX, is illustrated. AudioDirectionChangeX builds on the examples of FIGURES 4 and 5. AudioDirectionChangeX is defined for a Pos_Change event (i.e., a position change) and specifies the parameters to instantiate DIRSOUND, each parameter being public or protected. Further, the DIR CHANGE parameter of AudioDirectionChangeX is set to the difference between a newCoordinate and an oldCoordinate.

In view of the examples of FIGURES 7 and 8, rules can include different types of parameters, such as public and protected parameters. All values for public parameters need to be provided to apply the rule, and the protected parameters are derived from the context of the rule call and/or the public parameters. Further, parameters of the rules can be set based on external parameters. For example, in the example of FIGURE 7, density is an external parameter. As another example, in the example of FIGURE 8, newCoordinate and oldCoordinate are external parameters.

Building on the foregoing definition of a rule, it is also contemplated that a rule can identify an event and one or more patterns to link to the event. The patterns can be ordered in a sequence and can also loop. Further, for each pattern of the rule, the rule includes one or more parameters needed to instantiate the pattern. Hence, a rule can more generally be defined as:

| Event |
|---|
| Pattern Definition 1 |
| Pattern Definition 2 |
| ... |
| Pattern Definition N |

where Event identifies the event of the rule; N > 0; and each pattern definition i (0 < i < (N + 1)) identifies a pattern and defines the parameters to instantiate the pattern.

Referring back to FIGURE 1, the sensory generator system **10** further includes at least one processor **32** and at least program memory **34.** The program memory **34** includes processor executable instructions executed by the processor **32.** The sensory generator system **10** further includes a communication interface **36** and at least one system bus **38.** The communication interface **36** allows the processor **32** to interface with external devices and/or systems, such as the aural device **14** and the haptic device **12.** The system bus **38** interconnects the processor **32,** the program memory **34,** the storage memory **16** and the communication interface **36.**

The processor executable instructions include processor executable instructions embodying the software generator modules **20.** Further, the processor executable instructions include a rules engine **40.** The rules engine **40** receives event data from external systems, generated in response to trigger events, and executes corresponding rules in the rules database **30.** The event data includes the requisite data needed to execute the corresponding rules. For example, event data includes external parameter values and/or trigger event context.

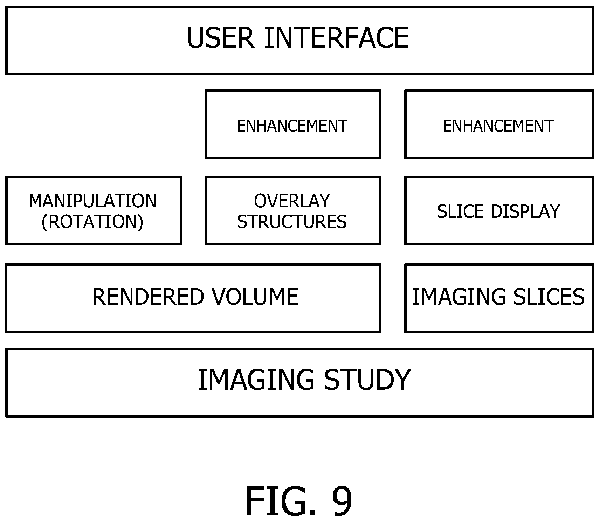

With reference to FIGURE 9, and continued reference to FIGURE 1, a medical system **50** enhanced with the sensory generator system **10** is provided. The medical system **50** includes an imaging system **52** to generate one or more images and/or maps of a region of a patient. Each of the images and/or maps describes at least one property, such as density, of the region. Further, each of the images and/or maps is typically three-dimensional (3D) and typically stored in an image and/or map memory **54** of the medical system **50.**

The imaging system **52** generates the images and/or maps using one or more imaging modalities, such as computed tomography (CT), positron emission tomography (PET), magnetic resonance (MR), MR spectroscopy (MRS), single photon emission computed tomography (SPECT), cone-beam computed tomography (CBCT), and the like. Hence, the imaging system **52** includes one or more scanners **56** corresponding to the imaging modalities, as well as one or more backend systems (not shown) acquiring raw image data from the scanners and reconstructing the raw image data into the images and/or maps. As illustrated, the imaging system **52** generates the images and/or maps using at least CT and includes a CT scanner **56.**

An image and/or map processing system **58** of the medical system **10** allows a user to analyze images and/or maps using one or more software tools **60.** The images and/or maps are typically received from the imaging system **52** and typically received by way of the image and/or map memory **54.**

The image and/or map processing system **58** includes at least one processor **62** and at least one program memory **64,** the program memory **64** including processor executable instructions executed by the processor **62.** The image and/or map processing system **58** further includes a communication interface **66** and at least one system bus **68.** The communication interface **66** allows the processor **62** to interface with external devices and/or systems. The system bus **68** interconnects the processor **62,** the program memory **64,** and the communication interface **66.**

The processor executable instructions embody the software tools **60.** Each of software tools **60** interface with the user with a graphical user interface (GUI). The GUI allows the user to control and/or otherwise interact with the tool. The graphical user interface displays graphical elements, such as icons, windows, menus, and so on, to a user on a display device **70.** The graphical user interface further allows the user to manipulate and/or otherwise interact with the graphical elements to control and/or otherwise interact with the tool using a user input device **72.**

The software tools **60** can segment images and/or maps to identify boundaries of relevant structures. The segmentation can be performed automatically and/or manually. As to automatic segmentation, a segmentation routine is employed to identify the boundaries of structures within an image and/or map. The segmentation routine can be one of any number of known segmentation routines, such as a model or atlas based segmentation routine. As to manual segmentation, an image and/or map is displayed to the user on the display device **70.** The user then uses the user input device **72** to identify the boundaries of the structures within the image and/or map.

It is also contemplated that the segmentation can be performed using a combination of automatic and manual segmentation. Namely, the boundaries of structures within an image and/or map can be automatically identified. The automatically identified boundaries can then be displayed to the user, optionally overlaid on the image and/or map, using the display device **70** and the user can modify the automatically identified boundaries, as necessary, using the user input device **72.**

The software tools **60** can also display images and/or maps to a user on the display device **70.** For 3D images and/or maps, typically only selected, 2D slices of the images and/or maps are displayed. In some embodiments, the boundaries of structures identified in the images and/or maps through segmentation can be highlighted in the images and/or images by overlaying contour lines on the images and/or maps. The user can further manipulate the images and/or maps with the user input device **72.** For example, the user can use the user input device **72** to select the displayed slice of a 3D image of a patient's heart.

The software tools **60** can also implement intervention functions. Intervention functions can include planning the trajectory of a shaft or needle, such as a catheter, from an entry point on the surface of a patient to a target of the patient using pre-procedure images and/or maps. Further, intervention functions can include monitoring the trajectory of the shaft or needle in real-time during intervention using, for example, the imaging system **52.** Even more, intervention functions can include displaying the current trajectory and, optionally, the planned trajectory overlaid on a pre-procedure image and/or map. Moreover, intervention functions can include displaying instructions to navigate the shaft or need to the planned trajectory.

The software tools **60** can also determine and display quantitative values for selected regions of displayed images and/or maps from the images and/or maps and/or additional data **74** received from external sources. For example, density values for selected regions of a displayed image and/or map can be determined from a corresponding density image and/or map and displayed. As another example, deviations between a parameter, such as volume, of a selected region of a patient's brain and the corresponding parameter for a normative model of a brain can be determined and displayed.

The sensory generator system **10** enhances the medical system **50** by adding one or more additional sensory channels with which to communicate with a user of the image and/or map processing system **58.** Namely, the image and/or map processing system **58** communicates with the user with sight. The sensory generator system **10** uses one or more additional senses, such as hearing and/or touch, to convey additional information. The sensory generator system **10** is invoked by the image and/or map processing system **58** through modification of the software tools **60** to generate event data for the sensory generator system **10.**

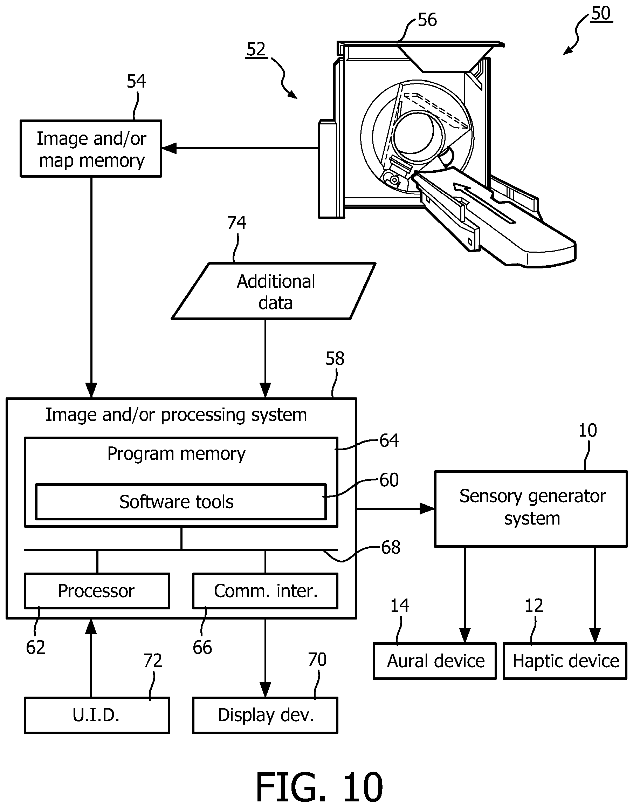

According to one enhancement, an additional sensory channel is added to convey data regarding displayed images and/or maps. Conceptually, in a radiology application, this is illustrated in FIGURE 10 in which the enhancement is added to existing modules for display images and/or maps. The data includes, for example, alerts, spatial orientation of the images and/or maps, properties of relevant parts of the images and/or maps, properties of data that is not visually available to the user (e.g., neural substrates, metabolites, etc.), data reinforcing user actions in a particular context, deviations from normative images and/or maps, deviations from template models, data derived from non-imaging studies, and so on.

The data can be obtained from and/or otherwise derived from one or more of: 1) the images and/or maps; and 2) the additional data **74,** such as normative images and/or maps, template models, data from non-imaging studies, and so on. Further, the data is obtained and/or otherwise derived by the image and/or map processing system **58,** for example, using the software tools **60.**

As an example, quantitative data, such as density, of various regions of an image and/or map displayed using the image and/or map processing system **58** can be conveyed aurally. To do so, one or more patterns are added to the pattern database **18** and annotated to aurally represent quantitative data. For example, the patterns of FIGURES 2 and 3 are added to the pattern database **18.** A rule is then added to the rules database **30** to configure an instance of the patterns based on quantitative value. For example, the rule of FIGURE 7 can be employed. The rule of FIGURE 7 modulates a base frequency using density. The rule is further configured for some event type, such as a MouseDown event, that the image and/or map processing system **58** is configured to monitor for.

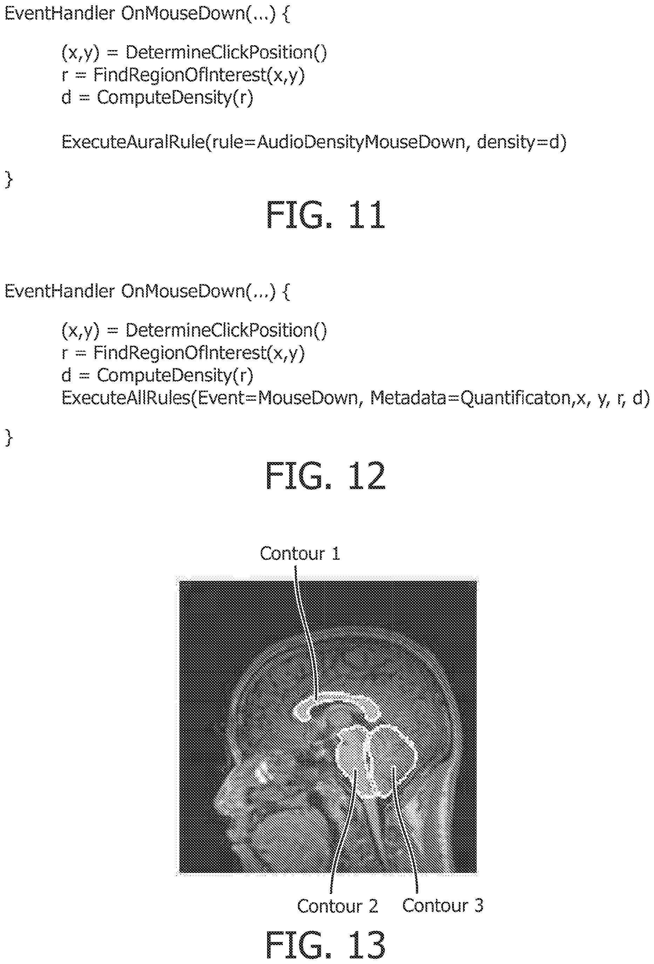

In response to an event corresponding to the rule within one of the various regions, the sensory generator system **10** receives event data, including a quantitative value for the region, from the image and/or map processing system **58.** The rules engine **40** executes the rule to generate an audio signal corresponding to the quantitative value of the event. The pseudo-code of FIGURES 11 and 12 can, for example, be employed by the GUIs of the software tools **60** to generate the event data. FIGURE 11 illustrates an example specifically instantiating the rule of FIGURE 7. FIGURE 12 illustrates an example in which all rules and patterns that match the current context are instantiated. This includes providing the coordinates and other local data to instantiate any rules that match.

As another example, suppose the image and/or map processing system **58** is employed to display a segmented image and/or map of a brain, as illustrated in FIGURE 13. The segmented image and/or map includes three contours, contour 1, contour 2, and contour 3, corresponding to three different regions of the brain. With a brain template model, degree of divergence of the various regions from the brain template model can be conveyed aurally using the sensory generator system **10.** As above, one or more templates and a rule are defined. The rule configures an instance of the patterns based on degree of divergence. In response to an event corresponding to the rule within the regions, the rules engine **40** executes the rule to generate an audio signal corresponding to the degree of divergence. As illustrated in FIGURE 13, the degrees of divergence of the three different regions are different, thereby yielding three different audio signals.

As another example, the amount of neurochemical metabolites can be conveyed through the additional sensory channel. MRS is the only imaging modality that can provide information on intracellular function and neurochemical composition in-vivo without the use of ionizing radiation. It shows promise as an imaging technique sensitive to detecting effects of various neurodegenerative disorders (e.g., Alzheimer's disease, Traumatic Brain Injury, etc.) including changes in neural integrity (e.g., reduced N-acetylaspartate levels - an amino acid synthesized in mitochondria that decreases with neuronal and axonal loss or dysfunction), brain energy metabolism (e.g., creatine), and membrane integrity/synthesis/repair (e.g., choline - primarily consisting of phosphoryl and glycerophosphoryl choline).

The sensory generator system **10** can be employed, through definition of appropriate rules and patterns, to modulate aural signals to represent the amount of neurochemical metabolites within a selected spectroscopic voxel and/or image region. The amount of neurochemical metabolites can be determined from an MRS image and/or map, and selection can be performed by, for example, moving a mouse cursor over, or clicking on, a voxel and/or image region in an image and/or map displayed by the image and/or map processing system **58.**

As another example, tissue integrity can be conveyed through the additional sensory channel. Diffusion tensor imaging (DTI) utilizes the properties of water diffusion to provide information about geometry and integrity of brain tissue. It is based on the principle that water molecules diffuse along the principal axes of their corresponding fiber tracts. The diffusion can be represented with a tensor centered at a voxel in the 3D image and/or map. The tensor describes the local rate of diffusion and can be visualized as an ellipsoid. As a result, voxels along common fiber pathways form "diffusion lines" (also called tracts), if viewed along the long axis of their individual tensors. DTI tractography is an image processing technique that traces such ellipsoids along their long axis by starting from a user defined position.

The sensory generator system **10** can be employed, through definition of appropriate rules and patterns, to modulate aural signals with the diffusion of selected tracts and their similarity to normative datasets. Selection can be performed by, for example, moving a mouse cursor over, or clicking on, tracts in an image and/or map displayed by the image and/or map processing system **58.** This could play an important role in the early diagnosis and longitudinal following of affected patients as disease progresses or is remediated with intervention.

As another example, functional impairment can be conveyed through the additional sensory channel. Functional MRI (fMRI) is a variation of MRI that utilizes the fact that oxygenated and deoxygenated haemoglobin have different magnetic properties and result in different MR signal intensity values. Tasks which increase regional brain activity and ultimately regional blood flow can be administered in the scanner. The subsequent changes in the ratio of oxygenated to deoxygenated blood can be used to generate images of task-related metabolic activity. This technique has been used to study cognitive function in individuals with neurodegenerative disorders.

The sensory generator system **10** can be employed, through definition of appropriate rules and patterns, to modulate aural signals with the ratio of oxygenated to deoxygenated blood within a selected brain region. Selection can be performed by, for example, moving a mouse cursor over, or clicking on, a brain region in an image and/or map displayed by the image and/or map processing system **58.**

According to another enhancement, an additional sensory channel is added to convey deviations between temporally disparate images and/or maps, such as an earlier reference image and/or map and a current image and/or map. Such deviations can include, for example, deviations in volume or area of a voxel and/or region, such as a nodule. To correlate voxels and/or regions in different images and/or maps, a registration routine is suitably employed. The software tools **60** suitably determine the deviations.

As an example, two images I₁ and I₂ of a region of a patient are acquired in a time span of 1 year. The sensory generator system **10** can be employed, through definition of appropriate rules and patterns, to modulate aural signals with the change in volume or area at present time compared to 1 year earlier within a selected region, such a nodule or other feature. Increased pitch, for example, may correspond to increased volume or area and decreased pitch to a decreased volume or area.

According to another enhancement, an additional sensory channel is added to facilitate navigation through displayed images and/or maps. The additional sensory channel can be used to indicate: 1) a navigation property while navigating through an image and/or map, such as direction of motion; or 2) a change in a navigation property while navigating through an image and/or map, such as a change of direction. The software tools **60** suitably determine the navigation properties and/or the changes.

For example, consider 3D images and/or maps, such as a 3D image and/or map of a brain, displayed using the image and/or map processing system **58.** As noted above, for 3D images and/or maps, typically only selected, 2D slices of the images and/or maps are displayed. The 2D nature of displayed slices can result in an increased need to verify and validate that the direction of navigation through the 3D images and/or maps is in the intended direction. Typically, this is performed through reference to a visualization of position indicators on the display device **70.** However, this can be difficult with an already overloaded sensory channel. An aural enhancement can be employed to indicate the direction of navigation through a 3D image and/or map and alleviate this difficulty.

To implement such an aural enhancement, one or more patterns are added to the pattern database **18** and annotated to aurally represent change in location information indicating directionality for multiple dimensions (e.g., three). For example, the patterns of FIGURES 4 and 5 are added to the pattern database. A plurality of rules, one for each dimension, is then added to the rules database. Each of the rules is configured to generate a different sound modulation pattern and is configured to vary to indicate direction of movement. For example, the rule of FIGURE 8 can be employed to indicate a change of position in the X direction by modulating a base frequency using change in position. Each of the rules is further configured for some event type, such as a Pos_Change event, that the image and/or map processing system **58** is configured to monitor for.

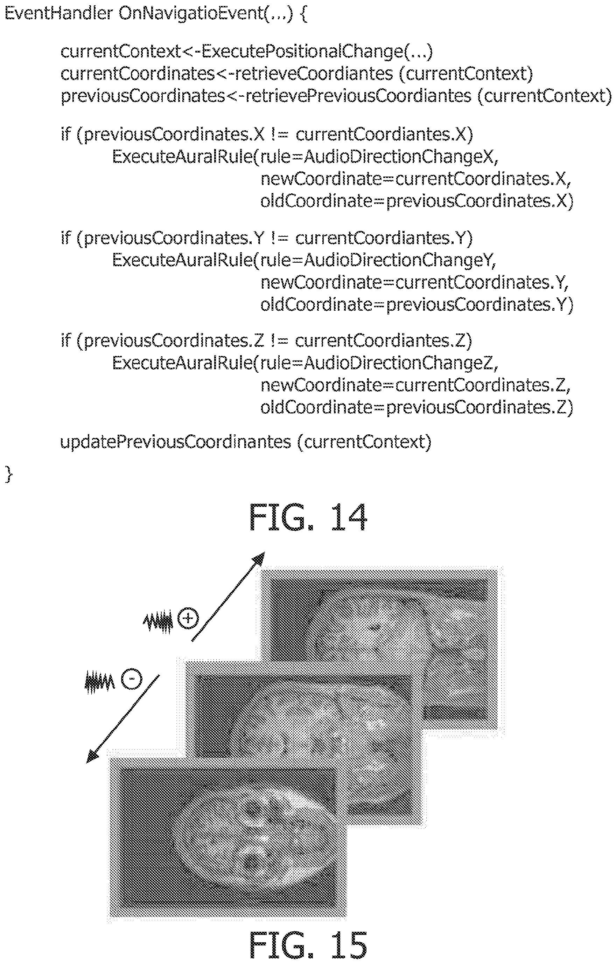

In response to an event corresponding to one of the rules (i.e., a change in position within the 3D image and/or map), the sensory generator system **10** receives event data, including a newCoordinate and an oldCoordinate, from the image and/or map processing system **58.** The rules engine **40** executes the rule to generate an audio signal corresponding to the change in position of the event. The pseudo-code of FIGURE 14 can, for example, be employed by the GUIs of the software tools **60** to generate the event data.

As another example, patterns and rules are established for each dimension of motion within 3D images and/or maps, similar to the previous example. The rules aurally indicate changes in the coordinates by generating increasing or decreasing pitch with a different base frequency for each dimension, as illustrated in FIGURE 15 for one dimension. For example, x-coordinates can be indicated using 200Hz (with a change of 20Hz), y-coordinates can be indicated using 300Hz (with a change of 30Hz), and z-coordinates can be indicated using 400Hz (with a change of 40Hz). The rules further include a sound panning rule for y-coordinates to further assist in user spatial orientation. This rule controls the side and degree of sound pan based on the change in the y-coordinate.

According to another enhancement, an additional sensory channel is added to facilitate intervention using displayed images and/or maps. The additional sensory channel can be used to indicate: 1) the direction of the needle or shaft; 2) the direction to navigate the needle or shaft to the target path; 3) potential obstacles in the path of the needle or shaft; 4) and so on. The software tools **60** suitably determine this data.

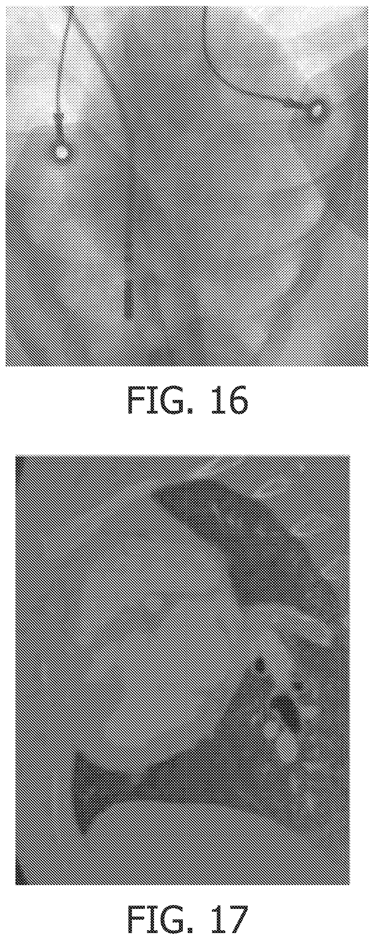

For example, consider minimally-invasive catheter-based ablation commonly used for many cardiac arrhythmias. Attempts to ablate complex arrhythmias, such as atrial fibrillation, are challenging because of limitations in fluoroscopic X-ray guidance. Interventional cardiac electrophysiology procedures are typically performed under fluoroscopy to visualize catheter location and device placement relative to highly-attenuating anatomical fiducial features, such as the spine or transiently-visible contrast-enhanced lumen structures. Fluoroscopy provides limited information about soft-tissue morphology and the true 3D shape of the anatomy of interest, as can be seen in the X-ray fluoroscopy projection of FIGURE 16. Furthermore, fluoroscopy times longer than one hour are not uncommon for complex procedures and result in significant X-ray exposure to the patient and clinicians.

Ablation strategies based on anatomical information from 3D images and/or maps, such as multislice computed tomography (MSCT) images and/or maps, an example of which is shown in FIGURE 17, can improve the efficacy of catheter ablation for these complex cases and reduce the X-ray dose significantly. Fusing information from pre-procedurally acquired 3D images and/or maps with the X-ray can provide clinicians with missing information about 3D shape and soft tissue morphology.

Rules and patterns can be built to implement sound modulation to guide and alert clinicians of potential obstacles in the path of the catheter or to identify proximity to adjacent anatomical structures. This would facilitate interventional catheter guidance relative to the 3D soft-tissue anatomy from the pre-procedurally acquired 3D images and/or maps. This can be extended to other types of guided surgery and other interventions where multiple layers of data are available to assess the position and navigation state and the delivery channels are overwhelmed or additional safety indications are benefitial.

According to another enhancement, an additional sensory channel is added to assess neurological disorders, such as neuroradiology and neurodegenerative disorders. The additional sensory channel can be used to indicate: 1) differences between healthy and diseased brain structures; 2) disease and/or abnormality; 3) organ specific information, such as local deformation of brain structures when compared to normative brain structures; 4) and the like. The software tools **60** suitably determine this data. The enhancement can further including sharing among different clinical centers over a network.

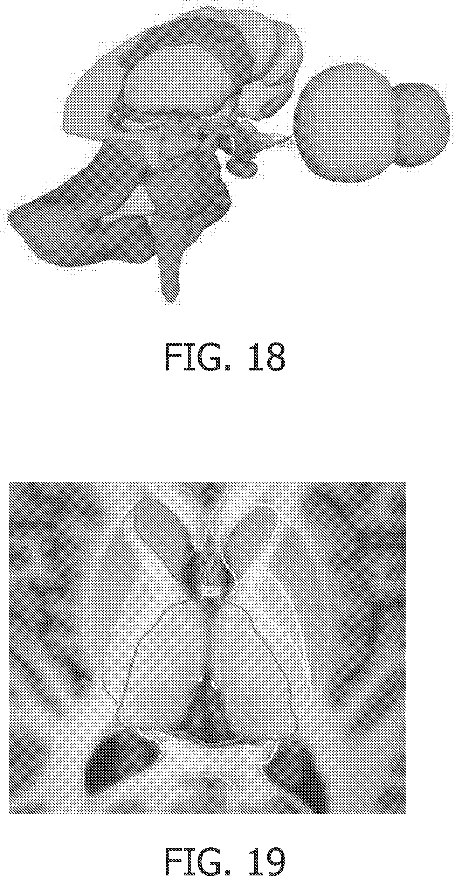

A normative dataset can be obtained by applying a routine for shape-constrained deformable segmentation of brain structures to a set of control patients and extracting a statistical representation of the underlined morphometry of brain structures. An example of a surface representation of a deformable brain model is illustrated in FIGURE 18, and an example of deformable brain model adapted to a patient's brain is illustrated in FIGURE 19. Coordinates, voxel values and different shape descriptors (e.g., surface curvature, point displacements from mid-sagittal plane, local deformation of surface, etc.) provide a quantitative description, which is straightforward to estimate. That would enable comparison of variances, biases, and other statistics with a certain confidence limit depending on the question being asked. Significance between groups or obtained results can also be established.

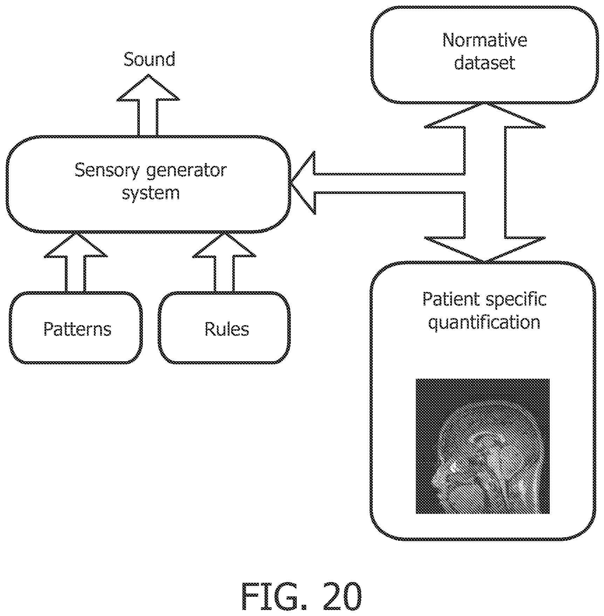

As an example of an enhancement assessing neurological disorders, suppose an additional aural sensory channel to convey deviations between brain structures and normative brain structures. The routine for shape-constrained deformable segmentation can be linked to the normative dataset using rules and patterns so that deviations between brain structures of a patient and normative brain structures are conveyed aurally. A flow chart illustrating this is shown in FIGURE 20.

One use case may involve a radiologist browsing through a patient's volume slice by slice. Segmentation, in this case, could be deployed in the background. As the user moves from one plane to another, patient specific values for displayed structures could be compared with a normative dataset. Based on the patterns and rules, the sensory generator system **10** could then present quantitative findings to the end user encoded as a sound. Quantitative values could represent volume, shape, neural substrates, etc. Furthermore, the location of a mouse cursor could be used to interrogate specific structures in a similar way.

Although this enhancement was presented in the context of neurological disorders, it applies to clinical applications in interventional and general radiology, neuropsychiatry, as well many other fields where the need to combine quantitative measurements and alert the end user for findings in an effective manner incorporated with their workflow still exists.

The sensory generator system **10** can be employed in in other clinical settings where the nature of the signals is different. In the above described uses of the sensory generator system **10,** the data was typically discrete in nature where the properties captured typically remained the same regardless of the point in time in which the data was explored. For example, features that are solely based on properties of a lung image and/or map characterize a nodule volume on the image and/or map. In other settings, for example, in intensive care and other applications of patient monitoring, the signals and the audio alerts vary based on the context.

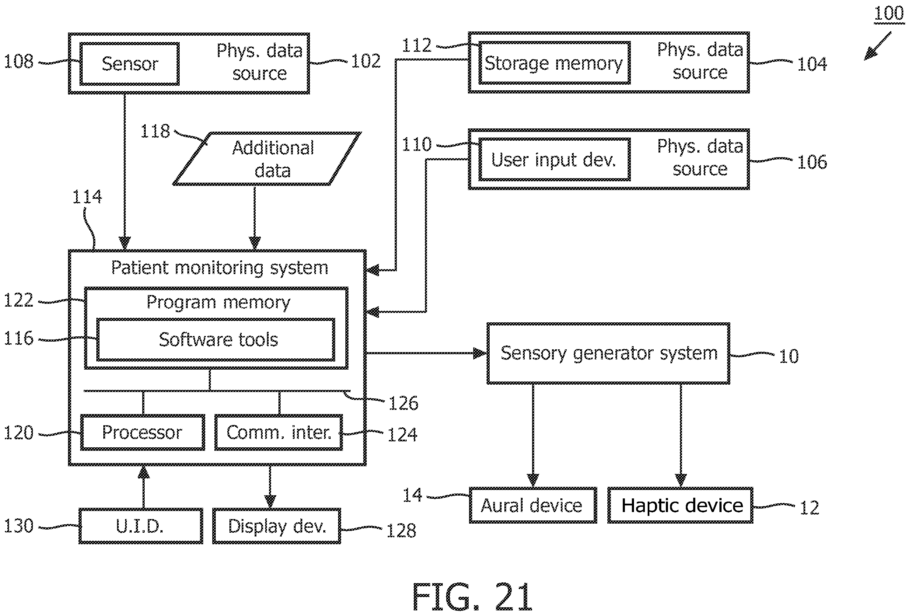

With reference to FIGURE 21, and continued reference to FIGURE 1, a medical system **100** enhanced with the sensory generator system **10** is provided. The medical system **100** includes one or more physiological data sources **102, 104, 106.** The physiological data sources **102, 104, 106** generate physiological data for corresponding patients and/or store physiological data for patients. The physiological data suitably includes data indicative of one or more physiological parameters, such as heart rate, temperature, blood oxygen saturation, level of consciousness, concern, pain, urine output, and so on. Examples of physiological data sources include, for example, patient monitors, nursing stations, mobile communications devices, patient information systems, and so on.

Each of the physiological data sources **102, 106** generating physiological data does so automatically and/or manually. As to the former, one or more sensors **108** of the physiological data source, such as electrocardiographic (ECG) electrodes, blood pressure sensors, SpO2 sensors, and so on, measuring physiological parameters can be employed. As to the latter, one or more user input devices **110** can be employed. Each of the physiological data sources **104** storing physiological data include one or more storage memories **112.**

A patient monitoring system **114** of the medical system **10** monitors one or more patients using corresponding physiological data and one or more software tools **116.** The physiological data is typically received from the physiological data sources **102, 104, 106.** Additional data **118,** such as normative datasets, received from external sources can also be employed for monitoring. The patient monitoring system **114** includes at least one processor **120** and at least one program memory **122,** the program memory **122** including processor executable instructions executed by the processor **120.** The patient monitoring system **114** further includes a communication interface **124** and at least one system bus **126.** The communication interface **124** allows the processor **120** to interface with external devices and/or systems. The system bus **126** interconnects the processor **120,** the program memory **122,** and the communication interface **124.**

The processor executable instructions embody the software tools **116.** Each of software tools **116** interface with the user with a graphical user interface (GUI). The GUI allows the user to control and/or otherwise interact with the tool. The graphical user interface displays graphical elements, such as icons, windows, menus, and so on, to a user on a display device **128.** The graphical user interface further allows the user to manipulate and/or otherwise interact with the graphical elements to control and/or otherwise interact with the tool using a user input device **130.**

The software tools **116** can display physiological data to a user on the display device **128** and allow the user to manipulate the physiological data with the user input device **130.** The software tools **116** can also generate alerts on, for example, the display device **128** if the physiological data meets alert criteria. For example, an alert can be generated if a patient's blood pressure exceeds a threshold. The software tools **116** can also derive additional physiological data, such as a stability index, from the physiological data.

The sensory generator system **10** enhances the medical system **100** by adding one or more additional sensory channels with which to communicate with a user of the patient monitoring system **100.** Namely, the patient monitoring system **100** communicates with the user with sight. The sensory generator system **10** uses one or more additional senses, such as hearing and/or touch, to convey additional information. The patient monitoring system **100** invokes the sensory generator system **10** through modification of the software tools **116** to generate event data for the sensory generator system **10.**

According to one enhancement, an additional sensory channel is added to convey data regarding alerts and/or physiological data received by, and/or generated by, the patient monitoring system **114** using the sensory generator system **10.** Rules and patterns are generated to produce sensory signals, such as auditory signals. These rules are defined based on characteristic features of the physiological data, such as heart rate, and based on the context of the measurement. For example, a "beep" signal that represents the heart rate of a patient can be modulated in two dimensions. One dimension depends on whether the heart rate is higher or lower than the expected heart rate, as defined based on the patient age, weight, etc. Another dimension depends on additional factors. For example, if a medication is introduced to the patient's system which is known to increase heart rate or decrease heart rate, different mechanisms are used to modulate in the first dimension to indicate the higher or lower normal heart rate.

Notwithstanding the sensory generator system **10** was described in the medical systems **50, 100** of FIGURES 9 and 12, the sensory generator system **10** and the use examples are applicable to other clinical scenarios, such as surgery, biopsy assistance, and beyond in domains such as meteorology, astronomy, and others. In more general, everyday use applications, the sensory generator system **10** can also be used to enhance or complement notifications in scenarios like driving or operating machinery, as well as in cases of navigation and interaction with complex data. Taking navigation, for example, sounds are modulated and transformed to complement the spoken instructions. For example, directions may be preceded with a sound alert that is modulated, panned, and transformed based on the proximity of the next trip node (e.g., higher pitch as an exit approaches, panning left for a left turn, etc.). In view of this, the sensor generator system **10** can be applied to any case where there is a need to combine data from multiple data modalities over the same point and also to capture some properties of the data, deliver analytics, and alert the user for focus points in the data.

Further, notwithstanding that the constituent systems of the medical systems **50, 100** of FIGURES 9 and 21 were discretely shown and described, it is to be appreciated that the constituent systems of the medical systems **50, 100** can be combined in any combination. For example, the sensory generator system **10** and the image and/or map processing system **58** of FIGURE 9 can be combined into a common system. As another example, the sensory generator system **10** and the patient monitoring system **114** can be combined into a common system. Moreover, while the sound generator **10** of FIGURE 1 was employed to enhance the medical systems **50, 100** of FIGURES 9 and 21, it is to be appreciated that additional and/or alternative sound generators can be employed. Even more, while the example applications of the sound generator **10** were described for sound, other sensors can be used with the examples.

As used herein, a memory includes one or more of a non-transient computer readable medium; a magnetic disk or other magnetic storage medium; an optical disk or other optical storage medium; a random access memory (RAM), read-only memory (ROM), or other electronic memory device or chip or set of operatively interconnected chips; an Internet/Intranet server from which the stored instructions may be retrieved via the Internet/Intranet or a local area network; or so forth. Further, as used herein, a processor includes one or more of a microprocessor, a microcontroller, a graphic processing unit (GPU), an application-specific integrated circuit (ASIC), an FPGA, and the like; a controller includes: (1) a processor and a memory, the processor executing computer executable instructions on the memory embodying the functionality of the controller; or (2) analog and/or digital hardware; a user input device includes one or more of a mouse, a keyboard, a touch screen display, one or more buttons, one or more switches, one or more toggles, voice recognition engines, and the like; a database includes one or more memories; and a display device includes one or more of a LCD display, an LED display, a plasma display, a projection display, a touch screen display, and the like.

The invention has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

## Claims

1. A medical system **(50, 100)** comprising:
at least one processor **(32, 62, 120)** programmed to:
receive patient-specific data of a patient, the patient-specific data including image and/or map data;
visually display at least some of the patient-specific data to a user of the medical system **(50, 100)** on a monitor **(70, 128);** and
frequency modulate an aural signal to convey quantitative data to the user using the sense of sound, the signal being modulated based on the quantitative data, the frequency modulating including modulating a pitch of the aural signal by increasing or decreasing the pitch to convey the quantitative data as additional information regarding displayed images and/or maps that is not visually available to the user in order to facilitate navigation of a needle or shaft through displayed images and/or maps and/or intervention using displayed images and/or maps, and
an aural device **(14)** for conveying the aural signal to indicate, while navigating the needle or shaft, one of
- a navigation property or a change in a navigation property,
- potential obstacles in the path of the needle or shaft
- proximity to adjacent anatomical structures.

2. The medical system **(50, 100)** according to claim 1, wherein the at least one processor is programmed to modulate the signal based on a first parameter extracted from the patient-specific data and/or a position of a device within the patient.

3. The medical system **(50, 100)** according to claim 2, wherein the at least one processor **(32, 62, 120)** is further programmed to:
display an image and/or map of the patient-specific data;
wherein the first extracted parameter corresponds to a selected region of the displayed image and/or map.

4. The medical system **(50, 100)** according to claim 3, wherein the first extracted parameter describes a relationship between multiple visual data points of the image and/or map data.

5. The medical system **(50, 100)** according to either one of claims 1 and 2, wherein
the at least one processor is further programmed to modulate the signal based on a second parameter extracted from normative patient data; and
the normative patient data includes one or more of a template model, an expected value for the parameter, and a normative data set.

6. The medical system **(50, 100)** according to any one of claims 1-5, further including:
a pattern database **(18)** including one or more patterns, each of the patterns defining a signal;
a rule database **(30)** including one or more rules, each of the rules linking one or more of the patterns to an event; and,
a rules engine **(40)** implemented by the at least one processor **(32, 62, 120),** the rules engine **(40)** instantiating at least one of the rules to modulate the signal.

7. The medical system **(50, 100)** according to claim 6, wherein one of the rules links a sequence of a plurality of the patterns to an event.

8. The medical system **(50, 100)** according to either one of claims 6 and 7, wherein one of the rules loops the patterns of the rule.

9. The medical system **(50, 100)** according to claim 1, wherein the at least one processor **(32, 62, 120)** is further programmed to:
display a selected slice of an image and/or map of the patient-specific data; and,
modulate the signal in accordance with change in position from a previously selected slice to a currently selected slice.

10. The medical system **(50, 100)** according to either one of claims 1 and 9, wherein the at least one processor **(32, 62, 120)** is further programmed to:
determine the position of the device within the patient; and,
modulate the signal in accordance with proximity of the device to a planned trajectory for the device and/or to anatomical structures of the patient.

11. At least one processor **(32, 62, 120)** programmed to perform a methodcomprising:
receiving patient-specific data of a patient, the patient-specific data including image and/or map data;
visually displaying at least some of the patient-specific data to a user of the medical system **(50, 100)** on a monitor **(70, 128);**
frequency modulating an aural signal to convey quantitative data to the user using the sense of sound, the signal being modulated based the quantitative data, the frequency modulating including modulating a pitch of the aural signal by increasing or decreasing the pitch to convey the quantitative data as additional information regarding displayed images and/or maps that is not visually available to the user in order to facilitate navigation through displayed images and/or maps and/or to determine navigation properties and/or changes and/or to facilitate intervention using displayed images and/or maps; and
conveying the aural signal to indicate, while navigating the needle or shaft, one of
- a navigation property or a change in a navigation property,
- potential obstacles in the path of the needle or shaft
- proximity to adjacent anatomical structures.

12. A non-transitory computer readable medium **(34, 64, 122)** carrying software which controls one or more processors **(32, 62, 120)** to perform a method comprising:
receiving patient-specific data of a patient, the patient-specific data including image and/or map data;
visually displaying at least some of the patient-specific data to a user of the medical system **(50, 100)** on a monitor **(70, 128);**
frequency modulating an aural signal to convey quantitative data to the user using the sense of sound, the signal being modulated based the quantitative data, the frequency modulating including modulating a pitch of the aural signal by increasing or decreasing the pitch to convey the quantitative data as additional information regarding displayed images and/or maps that is not visually available to the user in order to facilitate navigation through displayed images and/or maps and/or to determine navigation properties and/or changes and/or to facilitate intervention using displayed images and/or maps; and
conveying the aural signal to indicate, while navigating the needle or shaft, one of
- a navigation property or a change in a navigation property,
- potential obstacles in the path of the needle or shaft
- proximity to adjacent anatomical structures.

## Patentansprüche

1. Ein medizinisches System **(50, 100),** das Folgendes umfasst:
mindestens einen Prozessor **(32, 62, 120),** der für folgende Schritte programmiert ist:
Abrufen von patientenspezifischen Daten eines Patienten, wobei die patientenspezifischen Daten Bild- und/oder Kartendaten umfassen;
visuelles Anzeigen mindestens einiger der patientenspezifischen Daten für den Benutzer des medizinischen Systems **(50, 100)** auf einem Monitor **(70, 128);** und
Frequenzmodulieren eines akustischen Signals, um quantitative Daten mithilfe des Klangsinns an den Benutzer zu übertragen, wobei das Signal anhand der quantitativen Daten moduliert wird, und wobei die Frequenzmodulation das Modulieren der Tonhöhe des akustischen Signals durch Erhöhen oder Verringern der Tonhöhe umfasst, um die quantitativen Daten als zusätzliche Daten für die angezeigten Bilder und/oder Karten zu übermitteln, die dem Benutzer nicht visuell zur Verfügung stehen, und um die Navigation einer Nadel oder eines Schafts durch die angezeigten Bilder und/oder Karten und/oder einen Eingriff mithilfe der angezeigten Bilder und/oder Karten zu ermöglichen, und
ein akustisches Gerät **(14)** zum Übertragen des akustischen Signals, um während der Navigation der Nadel oder des Schachts eines der folgenden Elements anzuzeigen:
- eine Navigationseigenschaft oder eine Änderung einer Navigationseigenschaft,
- potentielle Hindernisse auf dem Weg der Nadel oder des Schafts
- die Nähe zu benachbarten anatomischen Strukturen.

2. Das medizinische System **(50, 100)** gemäß Anspruch 1, wobei der mindestens eine Prozessor das Signal beruhend auf einem ersten, aus den patientenspezifischen Daten extrahierten Parameter und/oder der Position eines Geräts im Patienten moduliert.

3. Das medizinische System **(50, 100)** gemäß Anspruch 2, wobei der mindestens eine Prozessor **(32, 62, 120)** zudem für folgenden Schritt programmiert ist:
Anzeigen eines Bilds und/oder einer Karte der patientenspezifischen Daten;
wobei der erste extrahierte Parameter einem ausgewählten Bereich des angezeigten Bilds und/oder der Karte entspricht.

4. Das medizinische System **(50, 100)** gemäß Anspruch 3, wobei der erste extrahierte Parameter eine Beziehung zwischen mehreren visuellen Datenpunkten der Bild- und/oder Kartendaten beschreibt.

5. Das medizinische System **(50, 100)** gemäß einem der Ansprüche 1 und 2,
wobei
der mindestens eine Prozessor das Signal zudem anhand eines zweiten, aus normativen Patientendaten extrahierten Parameters moduliert; und
wobei die normativen Patientendaten mindestens eines der folgenden Elemente umfasst: ein Schablonenmodell,
einen erwarteten Wert für den Parameter und einen normativen Datensatz.

6. Das medizinische System **(50, 100)** gemäß einem der Ansprüche 1 bis 5, das zudem Folgendes umfasst:
eine Musterdatenbank **(18)** mit mindestens einem Muster, wobei die einzelnen Muster jeweils ein Signal definieren;
eine Regeldatenbank **(30)** mit mindestens einer Regel, wobei die einzelnen Regeln mindestens eines der Muster mit einem Ereignis verknüpfen; und
ein Regelmodul **(40),** die vom mindestens einen Prozessor **(32, 62, 120)** implementiert wird, wobei das Regelmodul **(40)** mindestens eine der Regeln instantiiert, um das Signal zu modulieren.

7. Das medizinische System **(50, 100)** gemäß Anspruch 6, wobei eine der Regeln eine Sequenz mehrerer Muster mit einem Ereignis verknüpft.

8. Das medizinische System **(50, 100)** gemäß einem der Ansprüche 6 und 7, wobei eine der Regeln aus den Mustern der Regel eine Schleife bildet.

9. Das medizinische System **(50, 100)** gemäß Anspruch 1, wobei der mindestens eine Prozessor **(32, 62, 120)** zudem für folgende Schritte programmiert ist:
Anzeigen eines ausgewählten Ausschnitts eines Bilds und/oder einer Karte der patientenspezifischen Daten; und
Modulieren des Signals anhand der Veränderung der Position von einem zuvor ausgewählten Abschnitt in einen aktuell ausgewählten Abschnitt.

10. Das medizinische System **(50, 100)** gemäß einem der Ansprüche 1 und 9, wobei der mindestens eine Prozessor **(32, 62, 120)** zudem für folgende Schritte programmiert ist:
Ermitteln der Position des Geräts im Patienten; und
Modulieren des Signals anhand der Nähe des Geräts zur geplanten Bahn des Geräts und/oder zu anatomischen Strukturen des Patienten.

11. Mindestens ein Prozessor **(32, 62, 120),** der für eine Methode mit den folgenden Schritten programmiert ist:
Abrufen von patientenspezifischen Daten eines Patienten, wobei die patientenspezifischen Daten Bild- und/oder Kartendaten umfassen;
visuelles Anzeigen mindestens einiger der patientenspezifischen Daten für den Benutzer des medizinischen Systems **(50, 100)** auf einem Monitor **(70, 128);**
Frequenzmodulieren eines akustischen Signals, um quantitative Daten mithilfe des Klangsinns an den Benutzer zu übertragen, wobei das Signal anhand der quantitativen Daten moduliert wird, und wobei die Frequenzmodulation das Modulieren der Tonhöhe des akustischen Signals durch Erhöhen oder Verringern der Tonhöhe umfasst, um die quantitativen Daten als zusätzliche Daten für die angezeigten Bilder und/oder Karten zu übermitteln, die dem Benutzer nicht visuell zur Verfügung stehen, und um die Navigation durch die angezeigten Bilder und/oder Karten zu ermöglichen, und/oder um die Navigationseigenschaften zu ermitteln, und/oder um einen Eingriff mithilfe der angezeigten Bilder und/oder Karten zu ermöglichen, und
Übertragen des akustischen Signals, um während der Navigation der Nadel oder des Schachts eines der folgenden Elements anzuzeigen:
- eine Navigationseigenschaft oder eine Änderung einer Navigationseigenschaft,
- potentielle Hindernisse auf dem Weg der Nadel oder des Schafts
- die Nähe zu benachbarten anatomischen Strukturen.

12. Ein nicht flüchtiges, von einem Computer lesbares Medium **(34, 64, 122),** auf dem Software gespeichert ist, die mindestens einen Prozessor **(32, 62, 120)** steuert, um eine Methode durchzuführen, die folgende Schritte umfasst:
Abrufen von patientenspezifischen Daten eines Patienten, wobei die patientenspezifischen Daten Bild- und/oder Kartendaten umfassen;
visuelles Anzeigen mindestens einiger der patientenspezifischen Daten für den Benutzer des medizinischen Systems **(50, 100)** auf einem Monitor **(70, 128);**
Frequenzmodulieren eines akustischen Signals, um quantitative Daten mithilfe des Klangsinns an den Benutzer zu übertragen, wobei das Signal anhand der quantitativen Daten moduliert wird, und wobei die Frequenzmodulation das Modulieren der Tonhöhe des akustischen Signals durch Erhöhen oder Verringern der Tonhöhe umfasst, um die quantitativen Daten als zusätzliche Daten für die angezeigten Bilder und/oder Karten zu übermitteln, die dem Benutzer nicht visuell zur Verfügung stehen, und um die Navigation durch die angezeigten Bilder und/oder Karten zu ermöglichen, und/oder um die Navigationseigenschaften zu ermitteln, und/oder um einen Eingriff mithilfe der angezeigten Bilder und/oder Karten zu ermöglichen, und
Übertragen des akustischen Signals, um während der Navigation der Nadel oder des Schachts eines der folgenden Elements anzuzeigen:
- eine Navigationseigenschaft oder eine Änderung einer Navigationseigenschaft,
- potentielle Hindernisse auf dem Weg der Nadel oder des Schafts
- die Nähe zu benachbarten anatomischen Strukturen.

## Revendications

1. Système **(50, 100)** médical comprenant :
au moins un processeur **(32, 62, 120)** programmé :
pour recevoir des données spécifiques au patient d'un patient, les données spécifiques au patient comprenant des données d'image et/ou de carte ;
pour afficher visuellement au moins certaines des données spécifiques au patient à un utilisateur du système **(50, 100)** médical sur un moniteur **(70, 128)** ; et
pour moduler en fréquence un signal sonore pour transmettre des données quantitatives à l'utilisateur à l'aide du sens du son, le signal étant modulé en fonction des données quantitatives, la modulation en fréquence comprenant la modulation d'une hauteur tonale du signal sonore par augmentation ou par diminution de la hauteur tonale pour transmettre les données quantitatives en tant qu'informations supplémentaires concernant les images et/ou les cartes affichées, lesquelles ne sont pas visuellement disponibles pour l'utilisateur pour faciliter la navigation d'une aiguille ou d'une tige à travers des images et/ou des cartes affichées et/ou une intervention à l'aide des images et/ou des cartes affichées ; et
un dispositif auriculaire **(14)** destiné à l'acheminement du signal sonore pour indiquer, lors de la navigation de l'aiguille ou de la tige,
- une propriété de navigation ou un changement dans une propriété de navigation, et/ou
- des obstacles potentiels sur le chemin de l'aiguille ou de la tige, et/ou
- une proximité des structures anatomiques adjacentes.

2. Système **(50, 100)** médical selon la revendication 1, dans lequel l'au moins un processeur est programmé pour moduler le signal en fonction d'un premier paramètre extrait des données spécifiques au patient et/ou d'une position d'un dispositif à l'intérieur du patient.

3. Système **(50, 100)** médical selon la revendication 2, dans lequel l'au moins un processeur **(32, 62, 120)** est en outre programmé :
pour afficher une image et/ou une carte des données spécifiques au patient ;
dans lequel le premier paramètre extrait correspond à une zone sélectionnée de l'image et/ou de la carte affichée.

4. Système **(50, 100)** médical selon la revendication 3, dans lequel le premier paramètre extrait décrit une relation entre plusieurs points de données visuelles de l'image et/ou des données de carte.

5. Système **(50, 100)** médical selon l'une quelconque des revendications 1 et 2,
dans lequel
l'au moins un processeur est en outre programmé pour moduler le signal en fonction d'un second paramètre extrait des données normatives du patient ; et
les données normatives du patient comprennent un modèle et/ou une valeur attendue pour le paramètre et/ou un ensemble de données normatives.

6. Système **(50, 100)** médical selon l'une quelconque des revendications 1 à 5, comprenant en outre :
une base de données de motifs **(18)** comprenant au moins un motif, chacun des motifs définissant un signal ;
une base de données de règles **(30)** comprenant au moins une règle, chacune des règles liant l'au moins un des modèles à un événement ; et
un moteur de règles **(40)** mis en œuvre par l'au moins un processeur **(32, 62, 120),** le moteur de règles **(40)** instanciant l'au moins une des règles pour moduler le signal.

7. Système **(50, 100)** médical selon la revendication 6, dans lequel l'une des règles lie une séquence d'une pluralité de motifs à un événement.

8. Système **(50, 100)** médical selon l'une quelconque des revendications 6 et 7, dans lequel l'une des règles boucle les motifs de la règle.

9. Système **(50, 100)** médical selon la revendication 1, dans lequel l'au moins un processeur **(32, 62, 120)** est en outre programmé :
pour afficher une tranche sélectionnée d'une image et/ou d'une carte des données spécifiques au patient ; et
pour moduler le signal en fonction du changement de la position d'une tranche précédemment sélectionnée à une tranche actuellement sélectionnée.

10. Système **(50, 100)** médical selon l'une quelconque des revendications 1 et 9, dans lequel l'au moins un processeur **(32, 62, 120)** est en outre programmé :
pour déterminer la position du dispositif à l'intérieur du patient ; et
pour moduler le signal en fonction de la proximité du dispositif par rapport à une trajectoire planifiée pour le dispositif et/ou aux structures anatomiques du patient.

11. Au moins un processeur **(32, 62, 120)** programmé pour mettre en œuvre un procédé comprenant :
la réception des données spécifiques au patient d'un patient, les données spécifiques au patient comprenant des données d'image et/ou de carte ;
l'affichage visuel d'au moins certaines des données spécifiques au patient à un utilisateur du système **(50, 100)** médical sur un moniteur (**70, 128)** ;
la modulation en fréquence d'un signal sonore pour transmettre des données quantitatives à l'utilisateur à l'aide du sens du son, le signal étant modulé en fonction des données quantitatives, la modulation en fréquence comprenant la modulation d'une hauteur tonale du signal sonore par augmentation ou par diminution de la hauteur tonale pour transmettre les données quantitatives en tant qu'informations supplémentaires concernant les images et/ou les cartes affichées, lesquelles ne sont pas visuellement disponibles pour l'utilisateur pour faciliter la navigation à travers les images et/ou les cartes affichées et/ou pour déterminer les propriétés de navigation et/ou des modifications et/ou pour faciliter l'intervention à l'aide des images et/ou des cartes affichées ; et
la transmission du signal sonore pour indiquer, lors de la navigation dans l'aiguille ou dans la tige,
- d'une propriété de navigation ou un changement dans une propriété de navigation, et/ou
- des obstacles potentiels sur le chemin de l'aiguille ou de la tige, et/ou
- d'une proximité des structures anatomiques adjacentes.

12. Support non transitoire lisible par ordinateur **(34, 64, 122)** portant un logiciel, lequel commande au moins un processeur **(32, 62, 120)** pour mettre en œuvre un procédé comprenant :
la réception des données spécifiques au patient d'un patient, les données spécifiques au patient comprenant des données d'image et/ou de carte ;
l'affichage visuel d'au moins certaines des données spécifiques au patient à un utilisateur du système **(50, 100)** médical sur un moniteur **(70, 128)** ;
la modulation en fréquence d'un signal sonore pour transmettre des données quantitatives à l'utilisateur à l'aide du sens du son, le signal étant modulé en fonction des données quantitatives, la modulation en fréquence comprenant la modulation d'une hauteur tonale du signal sonore par augmentation ou par diminution de la hauteur tonale pour transmettre les données quantitatives en tant qu'informations supplémentaires concernant les images et/ou les cartes affichées, lesquelles ne sont pas visuellement disponibles pour l'utilisateur pour faciliter la navigation à travers les images et/ou les cartes affichées et/ou pour déterminer les propriétés de navigation et/ou des modifications et/ou pour faciliter l'intervention à l'aide d'images et/ou des cartes affichées ; et
la transmission du signal sonore pour indiquer, lors de la navigation dans l'aiguille ou dans la tige,
- d'une propriété de navigation ou un changement dans une propriété de navigation, et/ou
- des obstacles potentiels sur le chemin de l'aiguille ou de la tige, et/ou
- d'une proximité des structures anatomiques adjacentes.
